Europäisches Patentamt

European Patent Office

Office européen des brevets

(19)

(11) Publication number: **0 223 192 B1**

# EUROPEAN PATENT SPECIFICATION

(12)

(45) Date of publication of patent specification: **19.06.91**   (51) Int. Cl.⁵: **A61K 37/54**

(21) Application number: **86115665.1**

(22) Date of filing: **11.11.86**

The file contains technical information submitted
after the application was filed and not included in
this specification

(54) **Pharmaceutical composition having thrombolytic activity.**

(30) Priority: **11.11.85 NL 8503097**

(43) Date of publication of application:
**27.05.87 Bulletin 87/22**

(45) Publication of the grant of the patent:
**19.06.91 Bulletin 91/25**

(84) Designated Contracting States:
**AT BE CH DE ES FR GB GR IT LI LU NL SE**

(56) References cited:

**CHEMICAL ABSTRACTS, vol. 97, no. 9, 30th
August 1982, page 49, abstract no. 66201a,
Columbus, Ohio, US; O. MATSUO: "On the
thrombolytic properties of human tissue
plasminogen activator and urokinase", &
PROC. SERONO SYMP. 1982, 48, 55-61**

(73) Proprietor: **Leuven Research & Development
V.Z.W.
Benedenstraat 59A Groot Begijnhof
B-3000 Leuven(BE)**

Proprietor: **Collen, Désiré José
Schoonzichtlaan 20
B-3009 Winksele-Herent(BE)**

(72) Inventor: **Collen, Désiré José
Schoonzichtlaan 20
B-3009 Winksele-Herent(BE)**

(74) Representative: **Bruin, Cornelis Willem et al
Octrooibureau Arnold & Siedsma Sweelinck-
plein 1
NL-2517 GK The Hague(NL)**

**EP 0 223 192 B1**

**Description**

This invention relates to a thrombolytically active pharmaceutical composition which comprises a synergistic combination of known thrombolytic agents, as well as to the preparation and use of such a composition.

It is known that certain enzymes, called plasminogen activators, are capable of exerting a thrombolytic activity in vivo after intravenous administration to man or animal. Their activity is based upon activation of plasminogen, a blood constituent, which in its turn initiates a chain of reactions finally resulting into dissolution (lysis) of a fibrin-containing blood clot when such clot is present in a blood vessel. The total activation mechanism is rather complex and may show many differences depending on the type of plasminogen activator which has been used.

A distinction should be made between two types of plasminogen activator, viz. a tissue-type plasminogen activator (t-PA) and a urokinase-type plasminogen activator (u-PA). With regard to the latter a further distinction can be made between a two-chain form (referred to as urokinase) and a single-chain form (referred to as scu-PA). The activity mechanisms of these three plasminogen activators are different.

A plasminogen activator of the tissue-type (t-PA) is capable of causing plasminogen activation, but nearly exclusively in the presence of fibrin. This means that activation mainly occurs in the vicinity of a blood clot which is dissolved thereby, whilst virtually no activation of plasminogen occurs in the circulating blood. Such a plasminogen activator does not react with antisera against plasminogen activators of the urokinase-type.

The two-chain form of u-PA (referred to as urokinase) induces an efficient activation of plasminogen in blood, both in the presence and in the absence of fibrin. This means that blood clots can be dissolved but also that an activation of the fibrinolytic system in the circulating blood will occur which leads to fibrinogen break down and a bleeding tendency.

The single-chain form of u-PA (referred to as scu-PA) will activate plasminogen efficiently but only in the presence of fibrin-containing blood clots. Likewise as with t-PA, no activation of the fibrinolytic system in the circulating blood will occur notwithstanding the fact that the activation mechanisms are different and that scu-PA is immunologically unrelated to t-PA.

Among these three plasminogen activators, urokinase has been used in medical practice already for a long time and the described side effects have been felt as a handicap therein. The other two plasminogen activators are still in the stage of clinical experimentation although such experimentation is relatively far advanced in the case of t-PA.

During further research, it has now been found that t-PA and scu-PA and also t-PA and urokinase have a synergistic effect in vivo i.e. a higher thrombolytic activity than the activity of the agents separately, when they are simultaneously administered to man or animal. This implies that an equivalent or larger thrombolytic activity can be obtained with smaller doses than previously used, thus resulting into savings of expensive agents. Moreover, this implies that the side effects which mainly occur with urokinase (activation of the fibrinolytic system in blood and breakdown of fibrinogen) may largely or completely be avoided.

The invention is based upon these findings. In one aspect, it provides a thrombolytically active pharmaceutical composition comprising a synergistic combination of tissue-type plasminogen activator (t-PA) and a urokinase-type plasminogen activator (u-PA) as an active ingredient. Such combination may be either a combination of t-PA with scu-PA or a combination of t-PA with urokinase.

In another aspect, the invention provides a method of preparing a thrombolytically active pharmaceutical composition, comprising the step of combining a tissue-type plasminogen activator (t-PA) with a urokinase-type plasminogen activator (u-PA) in a pharmaceutically acceptable excipient or excipients. The resulting composition may have any appropriate form but is preferably in the form of an intravenous infusion fluid or a combination of such infusion fluids.

The tissue-type plasminogen activator to be used may be any conventional kind of t-PA, obtained from any suitable source. In the experiments to be described, the t-PA employed was recovered from the conditioned medium of a melanoma cell line, but recombinant t-PA, derived from transformed bacteria or cells will also be adequate.

The urokinase to be used is normally obtained from urine and will be commercially available.

The scu-PA to be used can in principle be obtained from several sources such as tissue cultures, urine and plasma, but preferably it is recovered from the culture medium of conditioned cell cultures or transformed bacteria.

Several methods are available for isolation and purification of the plasminogen activators from their media. Such methods usually involve chromatography in many steps.

The two types of plasminogen activator (t-PA and u-PA) may be combined in any suitable way.

2

Although it is possible to provide the combination as a two-component system, it is preferable to produce a one-component system in which both plasminogen activators are combined with each other in a common excipient. The excipient may be any medium which is conventional or suitable for this purpose.

The weight ratio between t-PA and u-PA in the invented composition may vary widely, for example in general between 1:10 and 10:1. A weight ratio between 1:2 and 2:1 is preferably used.

Pharmaceutical compositions containing a combination of plasminogen activators may have any form suitable for administration to man or animal. It is preferred, however, to have such compositions in the form of an intravenous infusion fluid or a combination of infusion fluids since this form offers the best chances for good results. Such infusion fluids or other composition forms may contain for instance about 0.2 mg/ml of plasminogen activator although in general concentrations ranging from 0.05 mg/ml to 10 mg/ml may be possible. Further, compositions having a higher concentration of plasminogen activator up to 100% may be made availabe provided that they are diluted to the just-mentioned range before utilisation.

The doses of t-PA and u-PA to be used when administering the pharmaceutical composition to a thrombosis patient may be significantly lower than required for each agent separately, due to the synergistic action of both agents. In general, such doses will be 5-30% and preferably 10-20 % of the effective dose of t-PA and u-PA when used separately. The invention is illustrated by the following experiments which should not be interpreted to restrict the invention.

Experiment I

This experiment demonstrates the effect of several plasminogen activators and combinations thereof on an experimental thrombus produced in rabbits.

In anesthesized white rabbits (New Zealand type) weighing 2.4 ± 3.0 kg, the external jugular vein was dissected over a length of 4 cm. A segment of the vein was isolated with the use of vascular clamps and its volume was determined. 0.1 ml thrombin solution (100 NIH-units per ml) was introduced into the emptied segment and followed by fresh blood mixed with 10-20 $\mu$l human fibrinogen labeled with radioactive iodine. The amount of blood corresponded to the predetermined volume of the vessel segment. A blood clot which formed in the vessel was allowed to age for 30 min before the vascular clamps were removed. Then, the amount of radioactivity in the vessel segment was determined.

Thereupon, several thrombolytic agents, namely t-PA, urokinase, scu-PA and combinations thereof were administered to the rabbits by infusion via a marginal ear vein. To this end, adequate amounts of the agents were diluted with a physiological saline solution to a final volume of 20 ml. The infusions continued over a period of 4 hours. 30 minutes thereafter, the vessel segment containing the thrombus was sutured at both ends and removed, whereupon the remaining radioactivity was determined. The degree of thrombolysis was calculated as the percent difference between the original radioactivity and the remaining radioactivity in the blood vessel segment.

A isotope balance was made by adding the radioactivity in the removed vessel segment and the radioactivity in the circulating blood at the end of the experiment and expressing the resulting value in percent of the original radioactivity in the vessel segment. Radioactivity in the thyroid and in the lungs was negligible.

During the infusion of the thrombolytic agents, and also before and after that period, several blood samples were taken for measurement of radioactivity, fibrinogen content, $d_2$-antiplasmin content and the content of t-PA and scu-PA.

For more details on the experimental model we refer to D. Collen et al., J.Clin.Invest. 73, (1983), 368-376. The t-PA used in these experiments was obtained from the conditioned cell culture medium of a melanoma cell line, as described by D.C. Rijken et al., J.Biol.Chem., 256 (1981 ), 7035-7041 and D. Collen et al., Thromb.Haemost. 48, (1982), 294-296. Urokinase was from a commercial source and scu-PA was obtained from the conditioned medium of a lung adenocarcinoma cell line as described by D.C. Stump et al., Thromb.Heamost. 54 (1985), 122.

The results of this experiment are illustrated in table 1 in which the reported values represent the mean of three tests. In the control groups, receiving solvents only, the mean extent of thrombolysis was 9 ± 1%.

From the table, it appears that thrombolysis was effected by systemic infusion of t-PA, urokinase or scu-PA and that the extent thereof was dose-dependent. Further, it appears that administration of urokinase in thrombolytic dose is associated with extensive fibrinogen breakdown.

Simultaneous administration of t-PA and scu-PA caused a comparable degree of thrombolysis at much lower doses. Simultaneous administration of t-PA and urokinase also had a synergistic effect on thrombolysis, but no synergism was observed between urokinase and scu-PA. None of the combined infusions induced systemic fibrinogen breakdown.

This indicates that a clear synergistic effect with regard to thrombolysis in vivo exists between t-PA and scu-PA and also between t-PA and urokinase. Combinations of these agents in doses of only 10-20 % of the thrombolytic doses of each agent separately induce already an equivalent or larger thrombolytic effect. Synergism was observed in weight ratios of t-PA to u-PA between 1:10 and 10:1 and was most significant at weight ratios between 1:2 and 2:1.

TABLE 1

| Dose (mg/kg) | | | Thrombolysis | Isotope reco- | Fibrinogen- |
|---|---|---|---|---|---|
| t-PA | scu-PA | Urokinase | (%) | very (%) | level (%) |
| 0 | 0 | 0 | 9 ± 1 | 99 ± 3 | 92 ± 11 |
| 0.05 | 0 | 0 | 14 ± 1 | 97 ± 2 | 113 ± 5 |
| 0.125 | 0 | 0 | 24 ± 1 | 89 ± 1 | 105 ± 1 |
| 0.25 | 0 | 0 | 32 ± 1 | 89 ± 2 | 93 ± 1 |
| 0.50 | 0 | 0 | 57 ± 6 | 83 ± 1 | 101 ± 3 |
| 0 | 0.20 | 0 | 13 ± 1 | 94 ± 1 | 92 ± 12 |
| 0 | 0.50 | 0 | 21 ± 1 | 94 ± 3 | 97 ± 3 |
| 0 | 1.0 | 0 | 31 ± 1 | 90 ± 1 | 92 ± 2 |
| 0 | 0 | 0.2 | 12 ± 1 | 99 ± 2 | 101 ± 5 |
| 0 | 0 | 0.5 | 15 ± 2 | 94 ± 2 | 95 ± 5 |
| 0 | 0 | 1.0 | 23 ± 1 | 95 ± 1 | 91 ± 7 |
| 0 | 0 | 2.0 | 44 ± 3 | 98 ± 5 | 37 ± 26 |
| 0.025 | 0.05 | 0 | 15 ± 1 | 97 ± 1 | 101 ± 2 |
| 0.05 | 0.10 | 0 | 23 ± 1 | 96 ± 4 | 99 ± 3 |
| 0.1 | 0.2 | 0 | 51 ± 10 | 88 ± 4 | 106 ± 1 |
| 0.025 | 0 | 0.1 | 17 ± 2 | 94 ± 3 | 96 ± 5 |
| 0.05 | 0 | 0.2 | 27 ± 2 | 90 ± 2 | 100 ± 4 |
| 0.10 | 0 | 0.4 | 39 ± 7 | 93 ± 1 | 102 ± 1 |
| 0 | 0.05 | 0.1 | 11 ± 2 | 95 ± 1 | 88 ± 14 |
| 0 | 0.1 | 0.2 | 17 ± 2 | 93 ± 1 | 99 ± 8 |
| 0 | 0.2 | 0.4 | 25 ± 2 | 92 ± 1 | 102 ± 2 |
| 0.012 | 0.10 | 0 | 18 ± 2 | 98 ± 2 | 100 ± 1 |
| 0.050 | 0.025 | 0 | 28 ± 2 | 95 ± 1 | 92 ± 3 |
| 0.10 | 0.025 | 0 | 27 ± 2 | 90 ± 2 | 81 ± 4 |
| 0.012 | 0.20 | 0 | 24 ± 1 | 94 ± 1 | 99 ± 6 |
| 0.025 | 0 | 0.20 | 29 ± 3 | 96 ± 1 | 98 ± 1 |
| 0.038 | 0 | 0.30 | 49 ± 7 | 93 ± 1 | 96 ± 9 |
| 0.050 | 0 | 0.10 | 25 ± 7 | 94 ± 3 | 106 ± 5 |

Experiment II

This experiment demonstrates the thrombolytic effect of synergistic combinations of plasminogen activators in human patients with acute myocardial infarction and coronary artery occlusion.

In all patients, selective right and left coronary arteriography was performed from the right brachial artery. Then, a combination of t-PA and u-PA (scu-PA or urokinase) diluted with physiological saline to a final volume of 100 ml was administered by intravenous infusion over a period of 60 min. Arteriograhy of the occluded coronary artery was repeated at 15 min intervals or when signs of reperfusion occurred. Blood samples were collected on citrate and fibrinogen was assayed immediately therein. The results are collected in table 2 wherein the headings of columns 3 and 4 have the following meanings:

time (1. time interval between on set of symptoms and start of the infusion,

time (2): time interval from start of infusion to reperfusion.

Reperfusion is defined as a complete filling of the coronary vessel distal to the original occlusion within a period of 3 cardiac cycles.

In a first study, a combination of 10 mg t-PA and 300,000 IV urokinase was administered to four patients with acute coronary occlusion (table 2A). In three of the four patients, reperfusion was obtained after 46, 50 and 53 minutes respectively. The fourth patient did not respond to this infusion and was also resistant to intracoronary streptokinase. None of the patients had any complications related to the infusion. The combined administration of t-PA and urokinase did not induce a systemic lytic state in any of the patients as evidenced by the unchanged levels of fibrinogen.

In a second study, a combination of 10 mg t-PA and 10 mg scu-PA was administered intravenously over one hour to seven patients having acute coronary occlusion (table B). A stable coronary reperfusion was obtained in five patients and a transient reperfusion in one patient (no. 4) whilst the remaining patient (no. 5) did not respond to the infusion. The combined infusion also did not induce fibrinogen breakdown here.

It may be concluded from table 2 that a combined administration of t-PA and u-PA to human patients will induce an efficient degree of thrombolysis, at doses significantly lower than normally used for each of the agents alone.

5

## Table 2

| Patient | Sex/Age | Time (1) (h) | Time (2) (min) | Fibrinogen level (%) |
|---|---|---|---|---|
| **A. 10 mg t-PA and 300,000 IU urokinase** | | | | |
| 1 | M/64 | 4.5 | none | 100 |
| 2 | M/63 | 4.0 | 50 | 100 |
| 3 | F/62 | 3.7 | 46 | 100 |
| 4 | M/69 | 4.6 | 53 | 100 |
| **B. 10 mg t-PA and 10 mg scu-PA** | | | | |
| 1 | M/66 | 1.8 | 23 | 88 |
| 2 | M/60 | 5.6 | 30 | 94 |
| 3 | M/65 | 5.7 | 60 | 100 |
| 4 | M/69 | 2.4 | 24 | 108 |
| 5 | M/58 | 6.0 | none | 115 |
| 6 | K/70 | 3.0 | 60 | 92 |
| 7 | M/51 | 5.5 | 45 | 33 |

## Claims

1. A pharmaceutical composition having thrombolytic activity and comprising a synergistic combination of tissue-type plasminogen activator (t-PA) and a urokinase-type plasminogen activator (u-PA) as active ingredients together with a pharmaceutically acceptable excipient or excipients, for simultaneous use in thrombolytic therapy.

2. A pharmaceutical composition as claimed in claim 1, wherein said synergistic combination is a combination of a tissue-type plasminogen activator (t-PA) with a urokinase-type plasminogen activator in one-chain from (scu-PA).

3. A pharmaceutical composition as claimed in claim 1, wherein said synergistic combination is a combination of a tissue-type plasminogen activator (t-PA) with a urokinase-type plasminogen activator in two-chain form (urokinase).

4. A pharmaceutical composition as claimed in any of claims 3, and comprising t-PA and u-PA in a weight ratio between 1:10 and 10:1.

5. A pharmaceutical composition as claimed in claim 4, wherein the weight ratio between t-PA and u-PA is between 1:2 and 2:1.

6. A pharmaceutical composition as claimed in any of claims 1-5, said composition being in the form of an intravenous infusion liquid or a combination of infusion liquids.

7. A method of preparing a thrombolytically active pharmaceutical composition, comprising the step of combining a tissue-type plasminogen activator (t-PA) with a urokinase-type plasminogen activator (u-PA) in a pharmaceutically acceptable excipient or excipients, for simultaneous use in thrombolytic therapy.

8. A method as claimed in claim 7, wherein said tissue-type plasminogen activator (t-PA) is combined with a urokinase-type activator in one-chain form (scu-PA).

9. A method as claimed in claim 7, wherein tissue-type plasminogen activator (t-PA) is combined with a urokinase-type plasminogen activator in two-chain form (urokinase).

10. A method as claimed in any of claims 7-9, wherein t-PA and u-PA are combined in a weight ratio between 1:10 and 10:1.

11. A method as claimed in claim 10, wherein t-PA and u-PA are combined in a weight ratio between 1:2 and 2:1.

12. A method as claimed in any of claims 7-11, wherein the resulting composition is in the form of an intravenous infusion fluid or a combination of intravenous infusion fluids.

13. Use of a synergistic combination of tissue-type plasminogen activator (t-PA) for preparing a pharmaceutical composition against thromboembolism.

Claims for the following Contracting States: GR,ES,AT

1. A method of preparing a thrombolytically active pharmaceutical composition, comprising the step of combining a tissue-type plasminogen activator (t-PA) with a urokinase-type plasminogen activator (u-PA) in a pharmaceutically acceptable excipient or excipients, for simultaneus use in thrombolytic therapy.

2. A method as claimed in claim 1, wherein said tissue-type plasminogen activator (t-PA) is combined with a urokinase-type activator in one-chain form (scu-PA).

3. A method as claimed in claim 1, wherein said tissue-type plasminogen activator (t-PA) is combined with a urokinase-type plasminogen activator in two-chain form (urokinase).

4. A method as claimed in any of claims 1-3, wherein t-PA and u-PA are combined in a weight ratio between 1:10 and 10:1.

5. A method as claimed in claim 4, wherein t-PA and u-PA are combined in a weight ratio between 1:2 and 2:1.

6. A method as claimed in any of claims 1-5, wherein the resulting composition is in the form of an intravenous infusion fluid or a combination of intravenous infusion fluids.

7. Use of a synergistic combination of tissue-type plasminogen activator (t-PA) and a urokinase-type plasminogen activator (u-PA) for preparing a pharmaceutical composition against thromboembolism.

**Revendications**

1. Composition pharmaceutique ayant une activité thrombolytique et comprenant une combinaison synergique d'un activateur du plasminogène du type tissulaire (t-PA) et d'un activateur du plasminogène du type urokinase (u-PA), en tant qu'ingrédients actifs, conjointement avec un excipient ou des excipients pharmaceutiquement acceptables, en vue d'une utilisation simultanée en thérapie thrombolytique.

2. Composition pharmaceutique selon la revendication 1, dans laquelle ladite combinaison synergique est une combinaison d'un activateur du plasminogène du type tissulaire (t-PA) avec un activateur du plasminogène du type urokinase sous une forme à une chaîne (scu-PA).

3. Composition pharmaceutique selon la revendication 1, dans laquelle ladite combinaison synergique est une combinaison d'un activateur du plasminogène du type tissulaire (t-PA) avec un activateur du plasminogène du type urokinase sous une forme à deux chaînes (urokinase).

4. Composition pharmaceutique selon l'une des revendications 1-3, et comprenant du t-PA et de l'u-PA dans un rapport en poids se situant entre 1:10 et 10:1.

5. Composition pharmaceutique selon la revendication 4, dans laquelle le rapport en poids entre le t-PA et l'u-PA se situe entre 1:2 et 2:1.

6. Composition pharmaceutique selon l'une des revendications 1-5, ladite composition se présentant sous la forme d'un liquide d'infusion intraveineuse ou d'une combinaison de liquides d'infusion.

7. Procédé de préparation d'une composition pharmaceutique ayant une activité thrombolytique, comprenant l'étape de combinaison d'un activateur du plasminogène du type tissulaire (t-PA) avec un activateur du plasminogène du type urokinase (u-PA) dans un excipient ou des excipients pharmaceutiquement acceptables, en vue d'une utilisation simultanée en thérapie thrombolytique.

8. Procédé selon la revendication 7, dans lequel ledit activateur du plasminogène du type tissulaire (t-PA) est combiné avec un activateur du type urokinase sous une forme à une seule chaîne (scu-PA).

9. Procédé selon la revendication 7, dans lequel ledit activateur du plasminogène du type tissulaire (t-PA) est combiné avec un activateur du plasminogène du type urokinase sous une forme à deux chaînes (urokinase).

10. Procédé selon l'une des revendications 7-9, dans lequel le t-PA et l'u-PA sont combinés dans un rapport en poids se situant entre 1:10 et 10:1.

11. Procédé selon la revendication 10, dans lequel t-PA et u-PA sont combinés dans un rapport en poids se situant entre 1:2 et 2:1.

12. Procédé selon l'une des revendications 7-11, dans lequel la composition résultante se présente sous la forme d'un fluide d'infusion intraveineuse ou d'une combinaison de fluides d'infusion intraveineuse.

13. Utilisation d'une combinaison synergique d'un activateur du plasminogène du type tissulaire (t-PA) et d'un activateur du plasminogène du type urokinase (u-PA) pour la préparation d'une composition pharmaceutique contre le thrombo-embolisme.

EP 0 223 192 B1

Revendication pour les Etats contractants suivants: GR, ES, AT

1. Procédé de préparation d'une composition pharmaceutique ayant une activité thrombolytique, comprenant l'étape de combinaison d'un activateur du plasminogène du type tissulaire (t-PA) avec un activateur du plasminogène du type urokinase (u-PA) dans un excipient ou des excipients pharmaceutiquement acceptables, en vue d'une utilisation simultanée en thérapie thrombolytique.

2. Procédé selon la revendication 1, dans lequel ledit activateur du plasminogène du type tissulaire (t-PA) est -combiné avec un activateur du type urokinase sous une forme à une seule chaîne (scu-PA).

3. Procédé selon la revendication 1, dans lequel ledit activateur du plasminogène du type tissulaire (t-PA) est combiné avec un activateur du plasminogène du type urokinase sous une forme à deux chaînes (urokinase).

4. Procédé selon l'une des revendications 1-3, dans lequel le t-PA et l'u-PA sont combinés dans un rapport en poids se situant entre 1:10 et 10:1.

5. Procédé selon la revendication 4, dans lequel le t-PA et l'u-PA sont combinés dans un rapport en poids se situant entre 1:2 et 2:1.

6. Procédé selon l'une des revendications 1-5, dans lequel la composition résultante se présente sous la forme d'un fluide d'infusion intraveineuse ou d'une combinaison de fluides d'infusion intraveineuse.

7. Utilisation d'une combinaison synergique d'un activateur du plasminogène du type tissulaire (t-PA) et d'un activateur du plasminogène du type urokinase (u-PA) pour la préparation d'une composition pharmaceutique contre le thrombo-embolisme.

**Ansprüche**

1. Pharmazeutische Zusammensetzung mit thrombolytischer Wirkung enthaltend eine synergistische Kombination eines Plasminogenaktivators vom Gewebetyp (t-PA) und eines Plasminogenaktivators vom Urokinase-Typ (u-PA) als Wirkbestandteile, zusammen mit einem oder mehreren pharmazeutisch verträglichen Exzipienten zur gleichzeitigen Anwendung für die thrombolytische Behandlung.

2. Pharmazeutische Zusammensetzung nach Anspruch 1, bei der die synergistische Kombination eine Kombination eines Plasminogenaktivators vom Gewebetyp (t-PA) mit einem Plasminogenaktivator vom Urokinase-Typ in einkettiger Form (scu-PA) ist.

3. Pharmazeutische Zusammensetzung nach Anspruch 1, bei der die synergistische Kombination eine Kombination eines Plasminogenaktivators vom Gewebetyp (t-PA) mit einem Plasminogenaktivator vom Urokinase-Typ in zweikettiger Form (Urokinase) ist.

4. Pharmazeutische Zusammensetzung nach jedem der Ansprüche 1 bis 3, mit einem Gehalt an t-PA und u-PA im Gewichtsverhältnis von 1:10 bis 10:1.

5. Pharmazeutische Zusammensetzung nach Anspruch 4, bei der das Gewichtsverhältnis von t-PA zu u-PA zwischen 1:2 und 2:1 liegt.

6. Pharmazeutische Zusammensetzung nach jedem der Ansprüche 1 bis 5, wobei die Zusammensetzung in Form einer intravenösen Infusionsflüssigkeit oder einer Kombination aus Infusionsflüssigkeiten vorliegt.

7. Verfahren zu Herstellung einer pharmazeutischen Zusammensetzung mit thrombolytischer Wirksamkeit, mit der Maßnahme, daß ein Plasminogenaktivator vom Gewebetyp (t-PA) mit einem Plasminogenaktivator vom Urokinase-Typ (u-PA) in einem oder mehreren pharmazeutisch verträglichen Exzipienten kombiniert wird, zur gleichzeitigen Anwendung für die thrombolytische Behandlung.

8. Verfahren nach Anspruch 7, bei dem der Plasminogenaktivator vom Gewebetyp (t-PA) mit einem

9

Aktivator vom Urokinase-Typ (scu-PA) in einkettiger Form kombiniert wird.

9. Verfahren nach Anspruch 7, bei dem der Plasminogenaktivator vom Gewebetyp (t-PA) mit einem Plasminogenaktivator vom Urokinase-Typ in zweikettiger Form (Urokinase) kombiniert wird.

10. Verfahren nach jedem der Ansprüche 7 bis 9, bei dem t-PA und u-PA in einem Gewichtsverhältnis von 1:10 bis 10:1 kombiniert werden.

11. Verfahren nach Anspruch 10, bei dem t-PA und u-PA in einem Gewichtsverhältnis von 1:2 bis 2:1 kombiniert werden.

12. Verfahren nach jedem der Ansprüche 7 bis 11, bei dem die resultierende Zusammensetzung in Form einer intravenösen Infusionsflüssigkeit oder einer Kombination aus intravenösen Infusionsflüssigkeiten erhalten wird.

13. Verwendung einer synergistischen Kombination aus einem Plasminogenaktivator vom Gewebetyp (t-PA) und einem Plasminogenaktivator vom Urokinase-Typ (u-PA) zum Herstellen einer pharmazeutischen Zusammensetzung gegen thrombotische Embolien.

Patentanspruch für folgende Vertragsstaaten: AT,ES,GR

1. Verfahren zu Herstellung einer pharmazeutischen Zusammensetzung mit thrombolytischer Wirksamkeit, mit der Maßnahme, daß ein Plasminogenaktivator vom Gewebetyp (t-PA) mit einem Plasminogenaktivator vom Urokinase-Typ (u-PA) in einem oder mehreren pharmazeutisch verträglichen Exzipienten kombiniert wird, zur gleichzeitigen Anwendung für die thrombolytische Behandlung.

2. Verfahren nach Anspruch 1, bei dem der Plasminogenaktivator vom Gewebetyp (t-PA) mit einem Aktivator vom urokinase-Typ (scu-PA) in einkettiger Form kombiniert wird.

3. Verfahren nach Anspruch 1, bei dem der Plasminogenaktivator vom Gewebetyp (t-PA) mit einem Plasminogenaktivator vom Urokinase-Typ in zweikettiger Form (Urokinase) kombiniert wird.

4. Verfahren nach jedem der Ansprüche 1 bis 3, bei dem t-PA und u-PA in einem Gewichtsverhältnis von 1:10 bis 10:1 kombiniert werden.

5. Verfahren nach Anspruch 4, bei dem t-PA und u-PA in einem Gewichtsverhältnis von 1:2 bis 2:1 kombiniert werden.

6. Verfahren nach jedem der Ansprüche 1 bis 5, bei dem die resultierende Zusammensetzung in Form einer intravenösen Infusionsflüssigkeit oder einer Kombination aus intravenösen Infusionsflüssigkeiten erhalten wird.

7. Verwendung einer synergistischen Kombination aus einem Plasminogenaktivator vom Gewebetyp (t-PA) und einem Plasminogenaktivator vom Urokinase-Typ (u-PA) zum Herstellen einer pharmazeutischen Zusammensetzung gegen thrombotische Embolien.